# EUROPEAN PATENT SPECIFICATION

(11) **EP 2 890 697 B1**
(45) Date of publication and mention of the grant of the patent: **06.09.2017**
(21) Application number: 13758972.7
(22) Date of filing: 05.07.2013
(51) Int. Cl.: C07K 1/06, C07K 9/00, A61K 31/7004, C12P 17/16, C12R 1/465

(54) **PSEUDOURIDIMYCIN (PUM) AND ITS DERIVATES**
PSEUDOURIDIMYCIN (PUM) UND SEINE DERIVATE
PSEUDO-URIDIMYCINE (PUM) ET SES DÉRIVÉS

(30) Priority: 09.08.2012 IT MI20121425
(43) Date of publication of application: 08.07.2015
(73) Proprietor: Naicons Srl, 20138 Milano (IT)
(72) Inventor: MAFFIOLI, Sonia, I-20127 Milano MI (IT); CANDIANI, Gianpaolo, I-20064 Gorgonzola (MI) (IT); GUGLIERAME, Paola, I-27010 Cura Carpignano (PV) (IT); MONCIARDINI, Paolo, I-21040 Cislago VA (IT); SERINA, Stefania, I-20162 Milano (MI) (IT); DONADIO, Stefano, I-21046 Malnate VA (IT)
(74) Representative: Trupiano, Federica
(86) International application number: PCT/IB2013/001473
(87) International publication number: WO 2014/024013

(56) References cited:
- EDUARDO KRAINER ET AL: "Synthesis and biological evaluation of dipeptidyl and tripeptidyl polyoxin and nikkomycin analogs as anticandidal prodrugs", JOURNAL OF MEDICINAL CHEMISTRY, vol. 34, no. 1, 1 January 1991 (1991-01-01), pages 174-180, XP055045809, ISSN: 0022-2623, DOI: 10.1021/jm00105a026

## Description

### State of the Art

Infections or infectious diseases occur as pathological reactions of the organism to the penetration and multiplication of micro-organisms such as viruses, bacteria, fungi, protozoa, metazoa, etc. Particularly, bacterial infections are infectious diseases caused by a bacterium passing from a source of infection to one or more susceptible individuals, i.e. individuals prone to contracting the infection itself. Antibiotics with antimicrobial/ antibacterial activity, i.e. substances able to counteract the bacterial infection, have been known for a long time. Over the years, the abuse and misuse of antibiotics have contributed to the emergence of resistant bacteria which have been shown to be non-responsive to the activity of an antibiotic drug, thereby generating a so-called "antibiotic resistance".

Known antibiotics act through different mechanisms of action and on different cellular targets.

Eduardo Krainer et Al., Journal of Medicinal Chemistry vol.34, n.1, Jan 1991, pages 174-180, discloses analogues of antibiotics nikkomycin and polyoxin, their synthesis and their biological properties against different strains of the pathogenic yeast *Candida albicans.* Polyoxines and nikkomycins, which inhibit chitin synthase, are potent competitive inhibitors of *C.albicans* in culture.

Some of the currently commercially available antibiotics target the RNA polymerase (RNAP) of bacteria. RNAP is an enzyme catalyzing the reaction of synthesis of a strand of RNA (RiboNucleic Acid) from DNA (DeoxyRibonucleic Acid). Several RNAPs exist depending on whether the organisms are prokaryotes or eukaryotes. Prokaryotic organisms, due to the simplicity of their genome, have only one, usually highly conserved RNAP, whereas eukaryotic organisms, due to the complexity of their genome, have three different types of RNAP, namely RNAP I, RNAP II and RNAP III. These genetic and structural differences between RNAPs of prokaryotes and eukaryotes result in that the bacterial - i.e. prokaryotic-type - RNAP represents an excellent target for drugs. In fact, the bacterial RNAP is a unique, highly conserved essential enzyme which is therefore a good target for a potentially broad-spectrum activity, whereas its differences from the RNAP existing in eukaryotes make it a selective target.

Two classes of antibiotic/ antibacterial drugs are known which target the bacterial RNAP: rifamycins (rifampicin, rifapentin, rifabutin and rifamixin) and fidaxomicin. Although they act on the same target, these two classes of molecules are not structurally related. Rifampicin is active on both Gram-positive and Gram-negative bacteria. Due to its broad-spectrum antimicrobial properties, rifampicin has been widely used over time to counteract bacterial infections, but such a use has generated bacteria in which the subunit of the enzyme is no longer available for binding to rifampicin. Therefore, these bacteria have become "resistant" to its action. Fidaxomicin is also an inhibitor of bacterial RNAP as described above, but it acts by binding an active site on the RNAP enzyme which is different from the site bound by rifamycins. In fact, whereas rifamycins block the RNAP enzyme through a steric effect, i.e. an effect related to the spatial distribution of the molecule with respect to the enzyme, fidaxomicin exerts a blockade through an allosteric effect, i.e. it can establish a reversible interaction (allosteric effect) with the enzyme which undergoes a conformational change (allosteric transition) such as to cause profound changes in the enzyme activity. Usually, rifamycin-resistant bacteria do not also show a cross-resistance to fidaxomicin, and vice versa.

Fidaxomicin is particularly active against Gram-positive bacteria, but its pharmacokinetic characteristics are such as to make it bioavailable only in the gastrointestinal tract while not being suitable to counteract an infection at the systemic level.

Hence, there is a need to find new classes of antibiotic agents with antimicrobial/ antibacterial activity which can overcome the limitations of the known art and which have a configuration and chemical-physical structure suitable to selectively interact with and inhibit the bacterial RNAP enzyme on sites other than those on which rifamycins and fidaxomicin act.

There is also a need to find new compounds which are antibiotics with a broad-spectrum antimicrobial/ antibacterial activity and able to reach different parts of the body without losing their antimicrobial efficacy.

Therefore, the present invention relates to novel compounds, the process for the preparation thereof, and the use thereof. These and other aspects and advantages therefrom will be better understood from the following description.

### DESCRIPTION OF THE INVENTION

The present invention relates to novel compounds of formula (I) 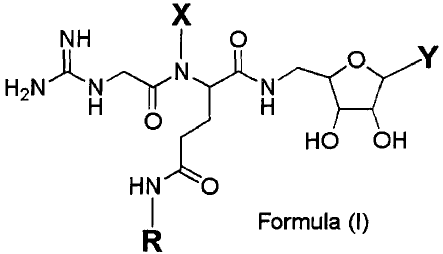 and pharmaceutically acceptable salts thereof.

The compounds of formula (I) are new products characterized by the presence of a pseudouridine group.

According to the present invention, and with reference to the general formula (I), R is independently selected from:
- H,
- a straight, branched, cyclic C₁-C₂₀ alkyl group, or combinations thereof,
- a straight, branched, cyclic C₂-C₂₀ alkenyl group, or combinations thereof;
- a straight, branched, cyclic C₂-C₂₀ alkynyl group, or combinations thereof;
- a benzylic group;
- a naphthylic group;
X is independently selected from H, OH, NH₂
Y is independently selected from one of the following heterocyclic groups:

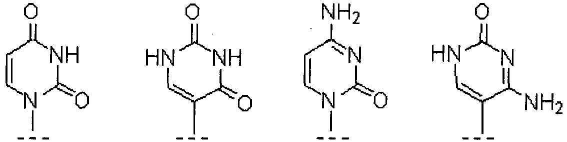

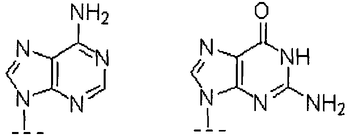

The invention also relates to optically pure compounds and stereoisomeric mixtures of the compounds of formula (I), for example mixtures of enantiomers and mixtures of diastereoisomers.

The stereocenters in the compounds of formula (I) may exist in the R and/or S configuration, unless otherwise indicated.

According to a preferred aspect of the invention, R is H, X is OH and Y is 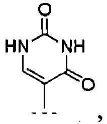 and the compound is represented by formula (II). The stereochemistry of the ribose is D, and that of the glutamine residue is L.

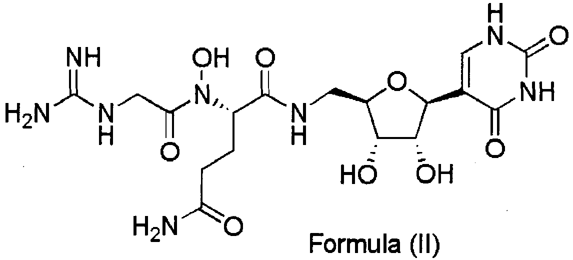

The compound of formula (II) according to the present invention is also referred to as Pseudouridimycin (PUM).

Another preferred aspect of the invention is represented by the compound of formula (III) 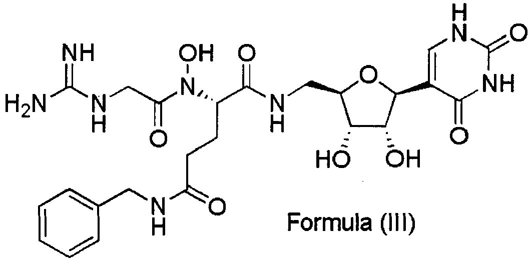 wherein, with reference to the general formula (I), X is selected to be equal to OH, R is selected to be equal to PhCH₂-, and Y is selected to be equal to 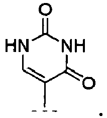 The stereochemistry of the ribose is D, and that of the glutamine residue is L. The preferred compound of formula (III) is also referred to as PUM benzylamide.

Still another preferred aspect of the invention is the compound of formula (IV) 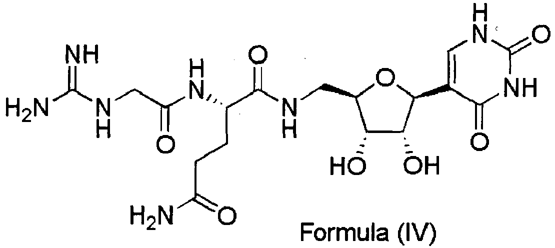 wherein, with reference to the general formula (I), X is selected to be equal to H, R is selected to be equal to H, and Y is selected to be equal to 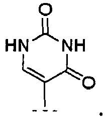 The stereochemistry of the ribose is D, and that of the glutamine residue is L.

The preferred compound of formula (IV) is also referred to as deoxy-PUM.

Yet another preferred aspect of this invention comprises the compound of formula (V), which can be obtained from the above-indicated compound of formula (II) (PUM) by mild acidic or basic hydrolysis as described later, and a semi-synthetic intermediate of certain products of formula (I).

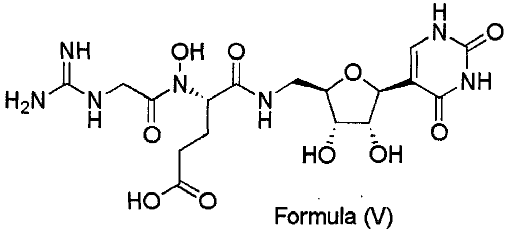

Surprisingly, the compounds of formula (I), which are characterized by the presence - never described heretofore in the state of the art for similar structures - of a pseudouridine derivative, have been shown to be inhibitors of RNAP. Due to their chemical-physical characteristics, and unlike the compounds described in the state of the art, the compounds according to the invention are characterized by the presence of nucleoside-type units within their structure, thus being able to be generally referred to as nucleoside analogues (NAI: Nucleoside Analogue Inhibitor). The compounds of formula (I) can surprisingly inhibit selectively the bacterial RNAP, and they are active against both Gram-positive and Gram-negative bacteria

The Experimental Section of the present specification provides details of comparative tests carried out to verify the selectivity and efficacy of the compounds of formula (I) compared to known reference compounds.

Another object of the present invention is the micro-organism *Streptomyces sp.* NAI38640 (deposited as number DSM26212 on July 20, 2012, in the Deutsche Sammlung von Mikroorganismen und Zellkulturen GmbH (DSMZ)) or a variant or mutant thereof capable of producing the compound of formula (II). The preferred compound of formula (II) according to the present invention is chemically characterized by the conjugation of a guanylated dipeptide of Glutamine-N-hydroxylated Glycine to 5'-amino-pseudouridine.

Experimental tests carried out on the compound of formula (II), also referred to as PUM, have shown that the compound itself, as well as the new class of compounds of formula (I) according to the invention to which it belongs, inhibit the bacterial RNAP of both Gram-positive and Gram-negative bacteria with an IC₅₀ (concentration producing 50% inhibition of enzyme activity) equal to 0.3-0.4 µM.

The compounds of the invention have a reduced or no inhibitory capacity against eukaryotic or phagic RNAP enzymes, thereby being selective for the bacterial enzyme.

Experimental tests carried out on the compound of formula (II), PUM, have shown that such a compound inhibits the growth of both Gram-positive and Gram-negative susceptible, resistant and multi-resistant pathogenic bacteria at concentrations in the range from 2 to 10 µg/ml. Other experimental tests have shown that PUM can treat an infection by *Streptococcus* in a murine model of peritonitis with an ED₅₀ of 10 mg/kg.

The present invention also relates to a semi-synthetic method for the preparation of compounds of formula (I), hereinafter also referred to as the process A, as well as to a fully-synthetic process for the preparation of the same compounds of formula (I), hereinafter also referred to as the process B.

Therefore, advantageously, the new compounds of formula (I) can be prepared alternatively according to either one of the processes A and/or B described hereinbelow.

### PROCESS A (SEMI-SYNTHESIS)

According to a preferred aspect of the invention, the semi-synthetic process A for the preparation of compounds of formula (I) comprises cultivating *Streptomyces sp.* NAI38640 (deposited as number DSM26212 on July 20, 2012 in the Deutsche Sammlung von Mikroorganismen und Zellkulturen GmbH (DSMZ)), or a variant or mutant thereof capable to produce a compound of formula (II), collecting the product of formula (II) from the mycelium and/or fermentation broth, isolating the pure compound of formula (II) by chromatographic techniques, and then modifying it by semi-synthesis.

### Production strain and fermentation

The production of the compounds of formula (I) via the semi-synthetic process A is obtained by cultivating a strain of *Streptomyces* capable of producing the product of formula (II), such as *Streptomyces sp.* DSM 26212 or a variant or mutant thereof which maintains the ability to produce the compound of formula (II). Then, this compound can undergo further modifications to give compounds belonging to the formula (I) as understood in its broadest sense. In a preferred aspect, the production of the compound of formula (II) is obtained under aerobic conditions in an aqueous production medium containing easily digestible and usable sources of carbon, nitrogen and inorganic salts, such as starch, dextrin, glucose, maltose and the like as the carbon source, soybean meal, peptone, meat extract, casein hydrolyzate, tryptone, yeast extract and the like as the nitrogen source. The medium can be eventually supplemented with salts capable of providing sodium, potassium, iron, zinc, magnesium, calcium, ammonium, chloride, carbonate, sulphate, phosphate, nitrate and the like ions.

The production strain for the compound of formula (II) is preferably grown in a flask or small fermenter, and the culture is used to inoculate fermentation reactors for the production. The pre-culture medium can be the same or different from that used for the production on an increased scale. According to a preferred aspect, *Streptomyces sp.* DSM 26212 is grown on S1 plates (see Experimental Section) in which the strain forms whitish colonies developing a grey aerial mycelium. The growth temperature for the strain *Streptomyces* sp. DSM 26212 is 26-35°C, preferably 28-32°C. During fermentation, the production of the compound of formula (II) is monitored by HPLC, and it generally occurs within 72 - 144 hours of fermentation.

### 16S rRNA gene sequence of Streptomyces sp. DSM 26212

SEQ ID NO 1 shows the partial sequence, consisting of 1441 nucleotides, of the gene encoding the 16S rRNA of the strain *Streptomyces* sp. DSM 26212. This sequence was compared with those deposited in public databases, and it was found to be highly related (>99.8%) to the 16S rRNA sequence of various strains of *Streptomyces (S. nigrescens, S. rimosus* subsp. *rimosus, S. tubercidicus, S. hygroscopicus* subsp. *angustmyceticus* and *S. libani* subsp. *Libani).*

As with other micro-organisms, the characteristics of the production strain for the compound of formula (II) can be mutated. For example, artificial variants and mutants of the strain can be obtained by treatment with known mutagenic agents such as UV rays, chemicals such as nitrous acid, N-methyl-N-nitrosoguanidine and others. All the natural or artificial variants and mutants of the strain *Streptomyces* sp. DSM 26212 can produce the compound of formula (II).
SEQ ID NO 1 (16S rRNA gene of the strain *Streptomyces* sp. DSM 26212)

### Isolation and purification

The compound of formula (II) is preferentially - although not exclusively - found in the clarified fermentation broth. The fermentation broth is then filtered and the mycelium separated and extracted as needed with a water-miscible solvent such as methanol, ethanol, propanol, acetone or the like. The extraction solution can then be combined again with the clarified broth. The isolation of the compound of formula (II) from the clarified broth is carried out by usual techniques including solvent extraction, precipitation by addition of non-solvents, forward phase-, reversed phase-, ion exchange- and molecular exclusion-chromatography, or a combination of these techniques. According to a preferred procedure, the clarified fermentation broth is contacted with an adsorption matrix and then eluted either with a mixture of water and water-miscible solvents or with buffered aqueous solutions. Examples of adsorption matrices are polystyrene resins or polystyrene-divinylbenzene resins (e.g. DOWEX 50WX2, M112 or S112, Dow Chemical Co.; Amberlite® XAD2 or XAD4, Rohm & Haas; Diaion HP 20, Mitsubishi), acrylic resins (e.g. XAD7 or XAD8, Rohm & Haas), polyamide resins (e.g. Polyamide-CC 6, Polyamide-SC 6, Polyamide-CC 6.6, Polyamide-CC 6AC and Polyamide-SC 6AC, Macherey-Nagel & Co.). Particularly, we prefer the use of resin DOWEX 50WX2 in conjunction with aqueous solutions which are buffered to the appropriate pH. If necessary, a subsequent purification of the product obtained can be carried out by chromatographic procedures which may include stationary phases such as silica gel, silanized silica gel or alumina which are eluted either with aqueous solvents or with mixtures of water and water-miscible solvents. For example, a reversed-phase chromatography with an RP-8 or RP-18 stationary phase and eluting phases based on either ammonium formate or dilute trifluoroacetic acid and water-miscible solvents such as acetonitrile or methanol can be used. The compound of formula (II) is then recovered by evaporation or lyophilization of the eluting solvents. As known in the state of art, the isolation and purification are monitored by analytical procedures such as HPLC or HPLC coupled to mass spectrometer.

### Semi-synthetic modification

Among the semi-synthetic modifications, an aspect of the present invention relates to the lengthening of the Glutamine chain of the compound (II) through a first step of hydrolyzing the primary amide which results in the formation of the compound of formula (V). The hydrolysis can be achieved by treatment with dilute acids or bases such as dilute HCl, trifluoroacetic acid, acetic acid or NaOH at room temperature. The compound of formula (V) can then be condensed with a suitable aliphatic or aromatic amine. The condensation can be achieved with the use of condensing agents such as dicyclohexylcarbodiimide (DCC)-hydroxybenzotriazole (HOBT), benzotriazolyl-oxy-tris-(dimethylamino)phosphonium fluorophosphate (HBTU), N,N,N',N'-tetramethyl-O-(benzotriazol-1-yl)uronium tetrafluoborate (TBTU), (O-(N-succinimidyl)-1,1,3,3-tetramethyluronium tetrafluoroborate (TSTU), benzotriazolyloxy-tris-(pyrrolidino)phosphonium hexafluorophosphate (PyBOP) in solvents such as DMF or N-methyl -pyrrolidone at temperatures ranging from 0° to 40°, preferably at room temperature.

The removal of the N-hydroxyl group can be obtained by reaction with a reducing agent such as Raney Nickel or RuCl₃ or TiCl₃. The reaction is preferably carried out at room temperature either in appropriately buffered water or in mixtures of water-methanol or water-ethanol, under an inert gas such as nitrogen or argon.

Optionally, this semi-synthetic step can be followed by a step of converting the compounds thus obtained into corresponding pharmacologically tolerated salts.

### PROCESS B (FULL SYNTHESIS)

According to another of its aspects, the invention relates to the fully-synthetic process B for the preparation of compounds of formula (I), which comprises condensing a protected dipeptide, which may be appropriately modified if needed, with a suitably protected aminonucleoside. Protected, suitably modified dipeptides are commercially available or easily obtainable by classical peptide synthesis and modifications thereof. The suitably 2',3'-protected aminonucleoside can be easily obtained from the corresponding commercial nucleoside by protection of the 2',3'-diol and replacement of the primary alcohol in 5' with sodium azide, followed by reduction to the amine. After the key step of condensation which characterizes the convergent synthesis, the removal of the protective groups and the guanylation result in PUM analogues according to formula (I).

More particularly, for example, the above fully-synthetic process B according to the present invention can be detailed as follows.
a) Protection of the 2',3'-diol of the nucleoside of formula (VI) by formation of the acetonide. The protection can be obtained by reaction with acetone or 2,2-dimethoxyacetone, alone or in the presence of a co-solvent such as, for example, DMF, and with the addition of an acidic catalyst selected, for example, from PPTSA (pyridinium-p-toluenesulfonic acid), PTSA (p-toluene sulfonic acid), HCl or H₂SO₄.
b) Activation for the nucleophilic substitution of the primary hydroxyl in position 5' by transformation into a suitable leaving group which can be selected, for example, from tosylate, mesylate and triflate, with mesylate (MsO) being preferable among them, to give the compound of formula (VII).
c) Nucleophilic substitution of the mesylate group (MsO) with NaN₃. The substitution reaction can be carried out in a solvent selected, for example, from DMF, acetonitrile, DMSO, and at a temperature ranging from 50 to 200°C, preferably of 100°C.
d) reduction of the azide group N₃ to a primary amine to give the compound of formula (VIII). The reduction can be obtained by reaction with an appropriate reducing agent such as, for example, hydrogen, in the presence of a Pt-or Pd-based catalyst or, for example, by reaction with phosphines such as, for example, Me₃P or Ph₃P.
e) Condensation of the amino group of the compound of formula (VIII) with the carboxyl group of the dipeptide of formula (IX) which is suitably protected on the amino group. The protection of the amino group of the dipeptide can be selected, for example, from t-butoxycarbonyl (Boc), carbobenzyloxy (Cbz) and 9-fluorenyl-methyl-carbamate (Fmoc), and it can be obtained by standard methods of peptide chemistry. The amidation can be achieved by addition of a condensing agent selected, for example, from dicyclohexylcarbodiimide (DCC) - hydroxybenzotriazole (HOBT), benzotriazolyl-oxy-tris-(dimethylamino)phosphonium fluorophosphate (HBTU), N,N,N',N'-tetramethyl-O-(benzotriazol-1-yl)uronium tetrafluoborate (TBTU), (O-(N-succinimidyl)-1,1,3,3-tetramethyluronium tetrafluoroborate (TSTU), benzotriazolyloxy-tris-(pyrrolidino)phosphonium hexafluorophosphate (PyBOP), in a solvent selected, for example, from DMF and N-methyl-pyrrolidone and at a temperature ranging from 0° to 50°C, preferably at room temperature.
f) Deprotection of the amino group of the peptide residue by standard methods suitable for the protective group of choice. For example, in the case of Fmoc, deprotection can be obtained by addition of piperidine in DMF at room temperature.
g) Transformation of the amino group into the guanidine group by reaction with 3,5-dimethylpyrazole-1 carboxyamidine.
h) Deprotection of the 2',3'-diol of the nucleoside according to step a) by treatment with weak acids such as mixtures of trifluoroacetic acid-H₂O or acetic acid-water to give the compound (I)

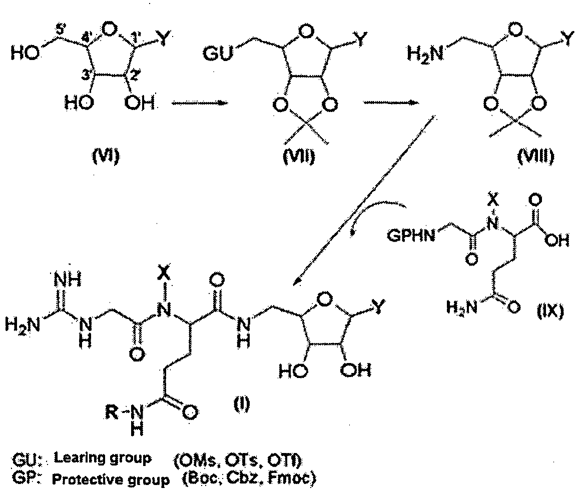

A particular case of the scheme described above allows for the synthesis of the product of formula (IV), and it is reported below. The reagents used are briefly reported below the scheme, while a detailed description of the experimental conditions is shown in the Example Section.

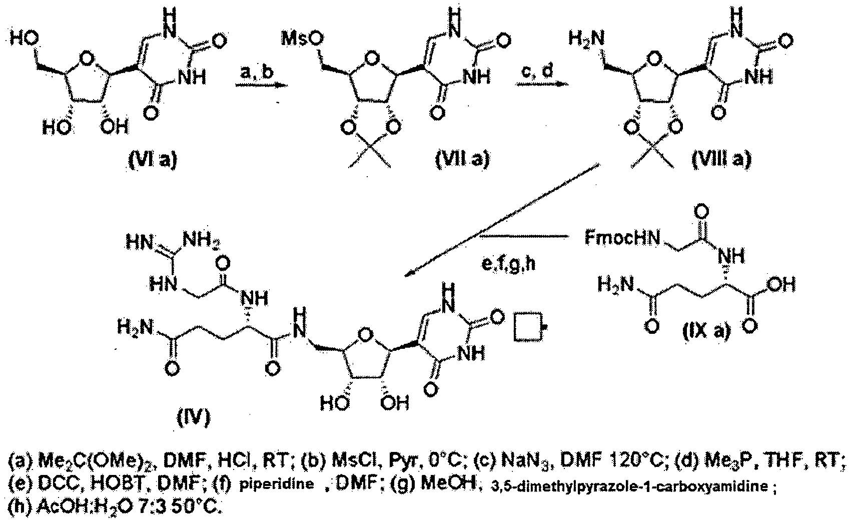

The present invention also relates to pharmaceutical compositions comprising the compounds of formula (I). The compounds of the present invention, in their pharmaceutically acceptable form, may be administered via oral, topical or parenteral route depending on the treatment to be performed. These compounds can be formulated into different dosage forms according to the route of administration. The preparations for oral administration may be in the form of capsules, lozenges, liquid solutions or suspensions. As known in the art, capsules and lozenges may contain usual excipients in addition to the active ingredient, for example extenders such as lactose, calcium phosphate, sorbitol and the like; lubricants such as magnesium stearate, polyethylene glycol (PEG), binding agents such as polyvinyl pyrrolidone, gelatine, sorbitol, acacia, flavoring agents, disintegrating agents and dispersing agents.

Liquid preparations, generally in the form of aqueous or oily solutions or suspensions, may contain conventional additives such as dispersing agents. For topical use, the compounds of formula (I) of the invention can also be prepared in suitable forms to be absorbed by either the mucous membranes of nose and throat or bronchial tissues, and they may advantageously be in the form of a spray. Topical applications can be formulated as ointments, lotions, gels or powders in hydrophobic or hydrophilic bases.

The present invention relates to compounds of formula (I) for use as a medicament. The present invention also relates to compounds of formula (I) for their use in the treatment of infectious diseases, particularly bacterial infectious diseases.

According to the present invention, compounds of formula (I) are used in the treatment of bacterial infectious diseases caused by Gram-positive and/or Gram-negative bacteria.

The compounds of the invention are generally active at doses in the range from 5 to 20 in weight per kg of body weight.

The compounds of this invention may also be used in combination with other drugs, such as other antibiotic agents or antibacterial/ antimicrobial agents. Therefore, the compositions and/or combinations and/or associations of the compounds of the present invention with other recognized, approved drugs fall within the purposes of the present invention.

The new compounds of formula (I) may be administered as they are or in admixture with pharmaceutically acceptable vehicles.

The present invention is better illustrated by means of the examples reported in the following, in no way being limitative.

### BRIEF DESCRIPTION OF THE FIGURES

FIGURE 1 shows the LC-MS (Liquid Chromatography-Mass Spectrometry) analysis of the compound of Formula (II) according to the invention, Pseudouridimycin (PUM).
FIGURE 2 shows the ¹H-NMR (¹H Nuclear Magnetic Resonance) spectrum of the compound of Formula (II) according to the invention, Pseudouridimycin (PUM), as recorded in dmso-d₆ at 25°C on a Bruker 400 MHz spectrometer.
FIGURE 3 shows the HSQC (Heteronuclear Single Quantum Correlation) spectrum of the compound of Formula (II) according to the invention, Pseudouridimycin (PUM), as recorded in dmso-d₆ at 25°C on a Bruker 400 MHz spectrometer.
FIGURE 4 shows the HMBC (Heteronuclear Multiple-Bond Correlation) spectrum of the compound of Formula (II) according to the invention, Pseudouridimycin (PUM), as recorded in dmso-d₆ at 25°C on a Bruker 400 MHz spectrometer.

### EXAMPLE 1. Fermentation, Isolation and Purification of (II) (Pseudouridimycin, PUM).

### Fermentation of Streptomyces sp. DSM 26212

The strain *Streptomyces* sp. DSM 26212 is grown on S1 plates for 2-3 weeks at 28°C [Composition of S1 (g/L): oat flakes 60, agar 18, FeSO₄ x 7 H₂O 0.001, MnCl₂ x 4 H₂O 0.001, ZnSO₄ x 7 H₂O 0.001. Oat flakes are boiled in 1 L of distilled water for 20 minutes and gauze-filtered. Then, they are added to the other components, the volume is brought to 1L with distilled water, and the pH is adjusted to 7.2 before sterilization at 120°C for 20 min.]. After a satisfactory growth is obtained, the micro-organism is recovered from the S1 plate and used to inoculate a 500-ml Erlenmeyer flask containing 100 ml of a vegetative medium having the following composition (g/L): dextrose monohydrate 20, yeast extract 2, soybean meal 8, NaCl 1 and calcium carbonate 4. The medium is prepared in distilled water and the pH is adjusted to 7.3 before sterilization at 121°C for 20 minutes. The inoculated flasks are incubated at 28°C on an orbital shaker at 200 revolutions per minute. After 2-3 days, this culture is inoculated at 5% into a second set of flasks containing the same medium. After 48 hours of incubation, 750 mL is transferred to a 19.5-L bioreactor containing 15L of a production medium having the following composition: (g/L): dextrose monohydrate 20, yeast extract 2, soy peptone 8, NaCl 1 and calcium carbonate 4. The medium is prepared in distilled water and the pH is adjusted to 7.3 before sterilization at 121°C for 25 min. Dextrose monohydrate is sterilized separately and added after cooling the bioreactor. The fermentation is carried out at 30°C under stirring at 600 rpm and aeration of 7.5 L per minute. The production of PUM is monitored by HPLC as described hereinbelow, and the culture is collected after 96 hours of fermentation.

### HPLC and LC-MS Conditions:

HPLC analysis is carried out on a Shimadzu instrument (LC-2010A HT, Shimadzu Corporation, Japan) equipped with a Waters Symmetry Shield RP8 5µ column (250 x 4.6 mm). Flow rate 1 ml/min. Gradient: time=0 (0% phase B); time=20 min (10% phase B); time=30 min (95% phase B); phase A is 2 mM heptafluorobutyric acid (HFBA) in water, and phase B is 2 mM HFBA acid in MeCN. UV detector at 230 nm and 260 nm. Under these conditions, PUM has a retention time of 10 min. HPLC-MS analysis is carried out on an Agilent HPLC 1100 instrument with a Waters Atlantis 50 x 4.6 mm 3 µm column eluted at 1 ml/min and maintained at 40°C. Gradient: time=0 (5% phase B); time=6 min (95% phase B); time=7 min (100% phase B). Phase A and phase B are 0.05% TFA (v/v) in water and acetonitrile, respectively. UV detector at 220 nm. The column flow is split in a 1:1 ratio; one portion is sent to the UV detector, and the other portion is sent to an ESI interface of a Bruker Esquire3000 Plus ion-trap mass-spectrometer. Under these conditions, PUM shows a retention time of 1.4 min. The mass analysis is carried out at the following conditions: sheath gas (N₂) 50 psi; dry gas 10 l/min; capillary temperature 365°C; positive polarity; capillary voltage -4000V; end plate offset - 500V; Scan conditions: maximum ion time 200 ms; ion time 5 ms; micro full scan 3, scan events positive (100-2400 m/z).

### Isolation and Purification of (II) (Pseudouridimycin, PUM)

The isolation and purification are monitored by HPLC or LC-MS according to the above methods. The fermentation broth (14 L) is filtered through a Buchner (Scienceware filter no. cat. 146320010). The filtered solution is then loaded onto a column containing a DOWEX 50WX2 resin (400 Mesh) (50 mL of resin / L of filtered solution). The flow of the column is maintained at 10 mL/min. The resin is then washed with the following buffer solutions: 5 column volumes (CV) of 20 mM AcONa, pH = 6, for glacial AcOH; 5 CV of 20 mM AcONa, pH 7, with 0.1 M NaOH, 2 CV of 100 mM AcONH₄, pH = 7, with 30% NH₄OH. Pseudouridimycin is then eluted with the following buffer solution: 6 CV of 100 mM AcONH₄, pH = 9, with 30% NH₄OH. The PUM-containing fractions are neutralized with a saturated solution of NaHCO₃ and evaporated to dryness. The semi-pure Pseudouridimycin thus obtained is purified by reversed-phase, medium-pressure chromatography: six chromatographic runs are carried out on a RediSep RF C18 86g column (40-63 µm particle size, 60A pore size, 230-400 mesh) with a Teledyne Isco CombiFlash RF chromatographic system. Phase A is water containing 0.02% trifluoroacetic acid (TFA), and phase B is acetonitrile. PUM is eluted with a linear gradient in which phase B is changed from 0 to 50% over 10 minutes. The PUM-containing fractions are combined again and concentrated under vacuum to obtain 1.5 g of PUM as a white solid.

### Physical and Chemical Characteristics of (II) (Pseudouridimycin, PUM)

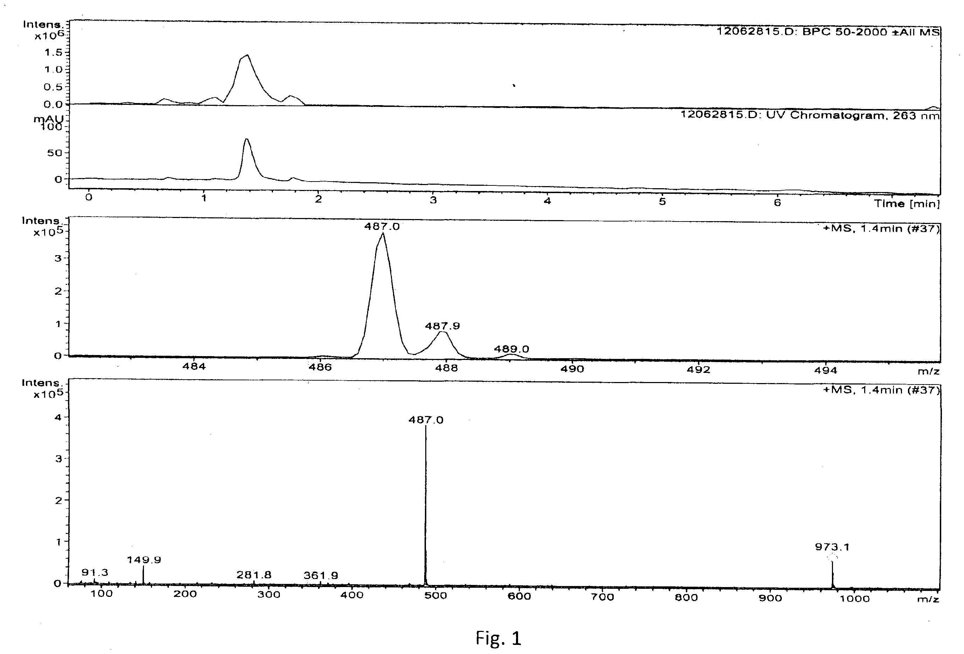

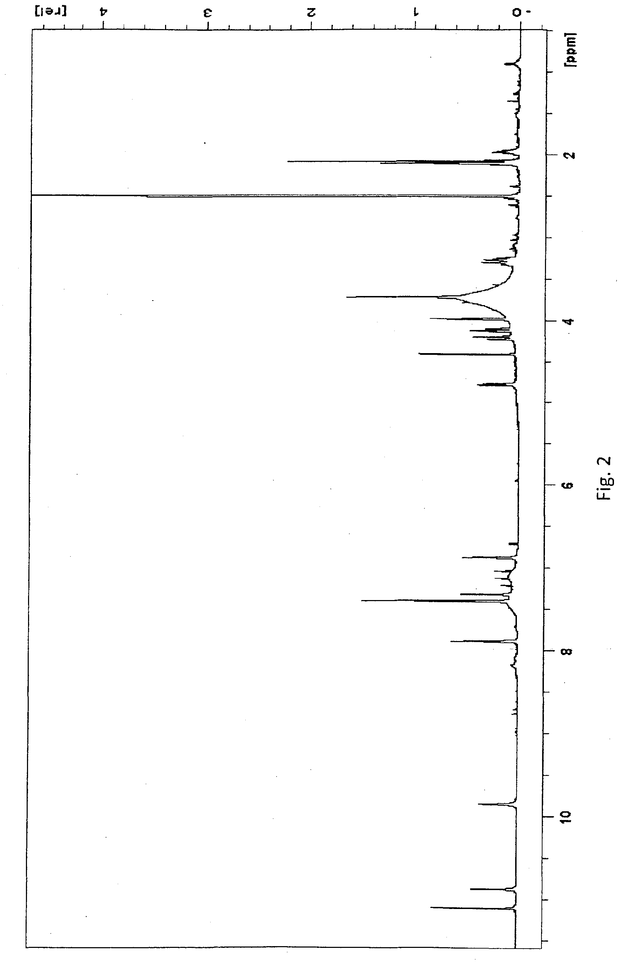

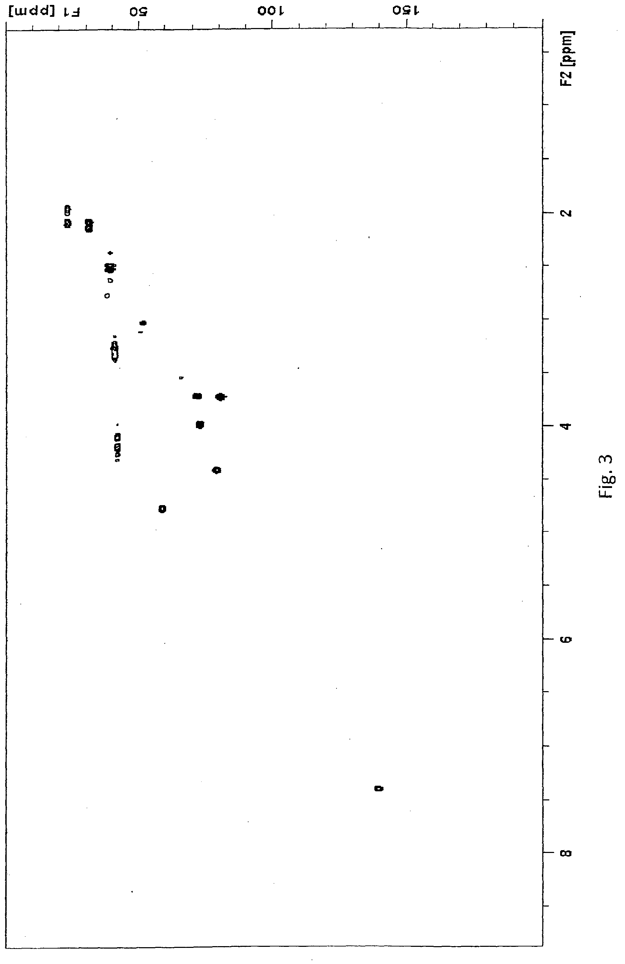

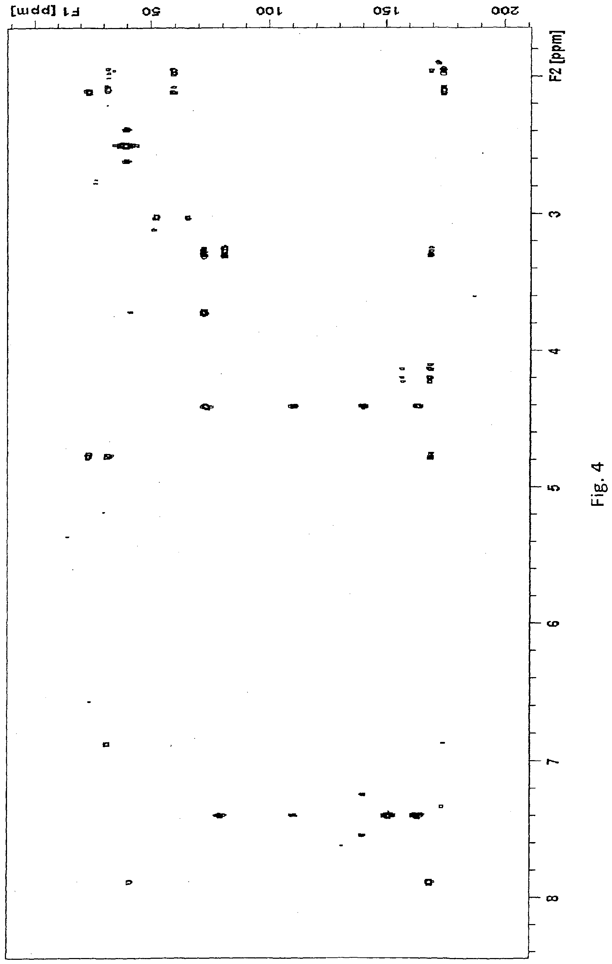

A) Mass spectrometry: Under the above LC-MS conditions, Pseudouridimycin shows a mono-charged ion at *m*/*z* 487 corresponding to [M+H]+, and a double-charged ion at *m*/*z* 973 corresponding to [2M+H]+. The LC-MS analysis is shown in figure 1.
B) The UV spectrum of PUM, obtained in 0.1% TFA with a Shimadzu Diode Array SPD-M10A VP detector (Shimadzu Corporation, Japan) during the HPLC analysis, shows an absorption maximum at 262 nm.
C) Mono- and two-dimensional 1H- and 13C-NMR experiments were recorded in dmso-d6 at 25°C on a Bruker 400 MHz spectrometer. If required, a sequence has been applied for suppressing the signal from water.
D) The ¹H-NMR spectrum of PUM, recorded in dmso-d₆, is shown in Figure 2 and exhibits the following signals (δ=ppm, dmso-d₆): 1.97 (m, 1H); 2.10 (m, 3H); 3.27 (m, 1H); 3.30 (m, 1H); 3.72 (m, 2H); 3.96 (m, 1H); 4.11 (d, J = 17.7 Hz, 1H); 4.21 (d, J = 17.7 Hz, 1H); 4.41 (d, J = 4.9 Hz, 1H); 4.78 (dd, 1H); 6.87 (broad s, 1H); 7.32 (broad s, 1H); 7.40 (m, 2H), 7.89 (t, J = 5Hz, 1H); 9.85 (broad s, 1H); 10.89 (broad m, 1H); 11.11 (broad m, 1H).
E) PUM also shows the following signals in the ¹³C analysis (δ=ppm, dmso-d₆): 23.6, 31.9, 41.9, 42.9, 59.5, 72.7, 73.6, 80.1, 81.2, 111.0, 140.5, 151.5, 157.0, 164.0, 168.9, 169.2, 174.0. The HSQC and HMBC spectra of PUM are shown in figures 3 and 4.
F) Determination of "acid-resistant" amino acids in PUM. PUM was completely hydrolyzed under acidic conditions (6N HCl at 105°C for 24 hours) and the hydrolyzed mixture was analyzed by GC-MS against a mixture of standard amino acids, thereby identifying the following amino acids: glycine and L-Asp.

### EXAMPLE 2: Biological activity of PUM

Effect on RNAP. The effect of PUM on bacterial RNAP is measured using an RNAP purified from either Escherichia coli (Sigma Aldrich) or *Bacillus subtilis* as described by Qi and Hulett (Qi Y, Hulett FM., Mol. Microbiol. 1998, 28(6):1187-1197). The bacterial RNAPs are used at 1.1 mg/ml, while the RNAP from bacteriophage T7 (Promega Corporation) is used at 20 U/ml. The nuclear extracts from HeLa cells (Human cervix carcinoma fibroblast; Promega Corporation) or NSO cells (non IgG secreting mouse myeloma lymphoblast; prepared as described by Dignam JD, Lebovitz RM, Roeder RG., Nucleic Acids Res. 1983, 11(5):1475-89) are used at 18 mg/ml. The reaction mixtures (50 µl) contain 40 mM Tris-HCl (pH 7.9), 6 mM MgCl₂, 2 mM spermidine, 10 mM NaCl, 10 mM DTT, 10 µg/ml bovine serum albumin, 100 µM ATP, CTP and GTP, 2 µM UTP, 0,5 µCi [α-³²P]UTP, PUM at the desired concentration, RNAP and template DNA. The templates used for the transcription are the plasmid pGEM3Z (20 nM; Promega Corporation) for the bacterial and phagic RNAPs, and DNA derived from calf thymus (20 µg/ml; Sigma Aldrich) for the eukaryotic polymerases. After 30 min. (bacterial and phagic RNAPs) or 60 min. at 37°C, the amount of radioactivity obtained after precipitation with trichloroacetic acid is measured as previously described (Mariani R, Granata G, Maffioli SI, Serina S, Brunati C, Sosio M, Marazzi A, Vannini A, Patel D, White R, Ciabatti R. Bioorg Med Chem Lett. 2005, 15(16):3748-3752). The results of these analyses are reported in Table 1.

**Table 1. IC50 values (µM) for RNAP inhibition by PUM, rifampicin (Rif) and fidaxomicin (Fdx).**

| Enzyme | PUM | Rif | Fdx |
|---|---|---|---|
| E. coli RNAP | 0.3 | 0.06 | 5.5 |
| B. subtilis RNAP | 0.4 | 0.05 | 0.9 |
| B. subtilis β(Q469R) | 0.1 | >121 | 0.7 |
| HeLa nuclear extract | >74 | nd | >92 |
| NSO nuclear extract | 205 | nd | 42 |
| T7 RNAP | >205 | >121 | nd |

As reported in Table 1, PUM inhibits RNAPs derived from Gram-negative and Gram-positive bacteria to the same extent without showing any cross-resistance with rifampicin.

Antibacterial activity. The antibacterial activity is determined by evaluating the effect on the growth kinetics as described (Holowachuka S, Bal'ab M, Buddington R., 2003, J. Microbiol. Meth. 55, 441-446). Micro-organisms are grown in either Todd Hewitt medium (*Streptococcus pyogenes)* or cation-adjusted Mueller Hinton Broth (*S. aureus, E. faecium, M. catarrhalis, E. coli, P. aeruginosa, S. maltophilia*) on 96-well microtiter plates. Each bacterial strain is inoculated with 5x10⁴ CFU/ml and incubated at 37°C on the Synergy 2 reader (BioTek), and the optical density is monitored at 595 nm for 48 h. The results are shown in Tables 2 and 3.

**Table 2. Inhibition of growth of streptococci by PUM, expressed as MIC (µg/ml). HS = Human Serum (+ presence 30% / - absence)**

| | HS | MIC |
|---|---|---|
| *Streptococcus pyogenes* L49 | - | 2 |
| *Streptococcus pyogenes* L49 | + | 2 |
| *Streptococcus pneumoniae* L44 | - | 3 |
| *Streptococcus pneumoniae* L44 | + | 2 |
| *Streptococcus pneumoniae* L899-Rif^{R} | - | 3 |
| *Streptococcus pneumoniae* L1407- Azi^{R} | - | 3 |
| *Streptococcus pneumoniae* ND061311- Pen^{R} Azi^{R} | - | 3 |
| *Streptococcus pneumoniae* L3909 - Pen^{R} Ery^{R} Chl^{R} Ctr^{R} | - | 2 |
| *Streptococcus pneumoniae* L1542 - Ami^{R} Ery^{R} Cli^{R} Gen^{R} Tet^{R} | - | 3 |
| *Streptococcus pneumoniae* L2868-8 MDR | - | 3 |

**Table 3. Inhibition of growth of Gram-negative bacteria by PUM, expressed as MIC (µg/ml).**

| | MIC |
|---|---|
| *Neisseria meningitidis* L1612 | 10 |
| *Haemophilus influenzae* L3296 | 0.8 |
| *Moraxella catarrhalis* L3294 | 0.8 |

As shown in Tables 2 and 3, PUM exhibits an antibacterial activity against susceptible, resistant and multi-resistant bacteria. PUM can also inhibit the growth of certain Gram-negative bacteria such as *Neisseria* sp., *Moraxella* sp., and *Haemophilus* sp. The antibacterial activity is not affected by the presence of human serum.

Effectiveness. The activity of PUM is demonstrated in experimental models of infection. ICR female mice (Harlan Italia) weighing 23-25 g are acclimated (23 ± 2°C, humidity, 55 ± 10% humidity) for one week before the experiment. The infection is induced by intraperitoneal injection of 4x10³ CFU of *S. pyogenes* C203 in 0.5 mL of saline containing 1% peptone. 48-72 h after infection, this inoculum leads to a mortality of at least 95% in untreated controls. Eight mice per group for each dose are treated with 0.25 mL of PUM prepared in 5% dextrose. Two different experiments were performed: in the first experiment, PUM is administered intravenously 10 min. after the infection and 6 hours later; in the second experiment, PUM is administered as a single intravenous or subcutaneous dose 10 min. after infection. The mortality of the animals is recorded daily. The ED₅₀ (50% effective dose) and 95% confidence limits are calculated as described (Finney, D.J. 1952. The Spearman-Kärber method. P. 524-30. In D.J. Finney (ed.) Statistical method in biological assay. Charles Griffin & Company Limited, London) for surviving animals at day 7 at each dose. The results are shown in Table 4.

**Table 4. ED₅₀ (mg/kg) of PUM in the S. pyogenes peritonitis model.**

| | ED₅₀ |
|---|---|
| iv: 10 min. and 6 h post-infection | 10 |
| iv: 10 min. post-infection | 20 |
| sc: 10 min. post-infection | 20 |

As shown in Table 4, PUM can treat the infection in a murine model of Streptococcus peritonitis with an ED₅₀ of 10 mg/kg. A comparable effectiveness is observed after intravenous and subcutaneous administration, thereby demonstrating that PUM, unlike fidaxomicin, is effective against systemic infections.

### EXAMPLE 3: Semi-synthesis of (V)

1st method: 4.5 mg of PUM (II) was dissolved in 2 mL of 1% trifluoroacetic acid in water. The transformation, which is monitored by HPLC, is complete after 96h. The solution is then neutralized by addition of a saturated solution of NaHCO₃ and the product is purified on a C18 reversed-phase silica cartridge to remove salts, thereby obtaining the compound of formula (V) after evaporation. MS analysis 488 [M+H]+ 2nd method: 10 mg of PUM (II) was dissolved in 1 ml of 0.01M HCl in water. The transformation, which is monitored by HPLC, is complete after 72h. The solution is then neutralized by addition of a saturated solution of Na₂CO₃ and then evaporated to yield the compound of formula (V).

### EXAMPLE 4: Semi-synthesis of (III) (PUM-Benzylamide)

2 mg of the product of formula (V) is dissolved in 1 ml of DMF to which 1.2 L of diisopropylethylamine (2 eq), 0.9 L of benzyl amine (2 eq) and 3.11 mg of HBTU are added. The reaction is allowed to stir for 1 hour at room temperature, and it is monitored by LC-MS until completion thereof. After neutralization with a solution of 1N HCl, the reaction mixture was used as is for the bioactivity tests. MS analysis 577 [M+H]+.

The compound III inhibits the bacterial RNAP with an IC₅₀ of 4 µM.

### EXAMPLE 5: Semi-synthesis of (IV) (Deoxy-PUM)

10 mg of PUM (II) was dissolved in 10 ml of 1M AcONa buffer, pH=7, and stirred under argon. A solution of TiCl₃, prepared by diluting 26 µL of a 10% by weight solution of TiCl3 in HCl (20-30%) in 500 L of water, is slowly dripped onto the solution. At the end of the addition, the reaction is allowed to stir continuously at room temperature for one hour. The solvent is then evaporated, and the product is purified by reversed-phase chromatography on an RP-8 silica column using 10% acetonitrile in a 0.2% aqueous solution of TFA as eluting phases. HPLC retention time 14 minutes (Shimadzu instrument and Symmetry Shield column according to the previously described method). MS m/z 471 [M+H]⁺. ¹H-NMR (400 MHz, dmso-d₆+D₂O, δ-H): 1.75 (m, 1H, Asn-β), 1.90 (m, 1H, Asn-β), 2.10 (m, 2H, Asn-γ), 3.29 (m, 2H, H-5'), 3.72 (m, 2H), 3.87 (broad s, 2H, Gly-α), 3.96 (m, 1H, H-2'), 4.24 (m, 1H, Asn-α), 4.40 (d, 1H, J=5.3 Hz, H-1'), 6.73 (broad s, CONH₂), 7.32 (broad s, CONH₂), 7.40 (s, 1H), 8.11 (broad t, 1H, NH), 8.34 (broad d, 1H, NH-Asn). ¹³C-NMR (dmso-d₆, δ-H): 28.4, 31.9, 41.4, 44.0, 53.0, 72.3, 73.7, 79.9, 81.6, 110.4, 141.5, 152.2, 158.2, 164.2, 168.0, 171.3, 173.7.

### EXAMPLE 6: Full synthesis of (IV) (Deoxy-PUM)

### Synthesis of (VII a):

From β-D-pseudouridine (VI a) to β-D-pseudouridine-2',3'-acetonide: A solution of β-D-pseudouridine (VI a) (400 mg, 1.64 mmol) in dimethylformamide (8 ml) and 2,2-dimethoxymethane (12 ml) is added with concentrated HCl, and the reaction mixture is allowed to stir at room temperature for 5 hours. After neutralization with 2.5 M NaOH, the solvent is evaporated under vacuum, and the crude product is used for the next step without further purification. ¹H-NMR for β-D-pseudouridine-2',3'-acetonide (400 MHz, D2O, δ-H): 1.35 (s, 3H, CH₃), 1.56 (s, 3H, CH3), 3.67 (dd, 1H, J = 12.2, 5.65 Hz, H-5'), 3.75 (dd, 1H, J = 12.2, 3.75 Hz, H-5'), 4.11 (dd, 1H, H-4'), 4.75 (m, 2H), 4.86 (m, 1H), 7.62 (s, 1H, pseudouridine).

From β-D-pseudouridine-2',3'-acetonide to (VII a): A solution of β-D-pseudouridine-2',3'-acetonide (419 mg) in pyridine (4.7 mL) is added with MsCl (95 µL) at 0°C, and the reaction solution is allowed to stir at room temperature for 16 hours. µ The solvent is then removed by evaporation under reduced pressure, and the crude mesylate is purified by a forward-phase, medium-pressure CombiFlash chromatograph (Teledyne ISCO) to give 475 mg of (VIIa) as a white powder. 95% yield. ¹H-NMR (400 MHz, MeCN-d₃, δ-H): 1.32 (s, 3H, CH₃), 1.54 (s, 3H, CH₃), 4.33 (dd, 1H, J = 11 Hz, H-5'), 4.46 (dd, 1H, J = 11 Hz, H-5'), 4.20 (m, 1H), 4.72 (dd, 1H), 4.80 (m, 2H) 7.55 (s, 1H, H-6), 10.23 (sb, 1H, NH), 10.45 (sb, 1H, NH).

### Synthesis of (VIII a):

Azidation of (VII a): A solution of (VII a) (475 mg) in DMF (24 ml) is added with NaN₃ (476 mg) and the reaction mixture is heated to 100°C over 4 hours, after which time the reaction is complete. The solvent is then evaporated under reduced pressure, and the crude azide is used for the next step without further purification. ¹H-NMR (400 MHz, MeCN-d₃, δ-H): 1.30 (s, 3H, CH₃), 1.50 (s, 3H, CH₃), 3.52 (d, 2H, J = 5.3 Hz, H-5'), 4.04 (m, 1H, H-3'), 4.69 (dd, 1H, H-4'), 4.75 (d, 1H, J = 3.3 Hz, H-1'), 4.87 (dd, 1H, J = 3.3 Hz, H-2') 7.58 (s, 1H, H-6).

Reduction of the azide to give (VIII a): A solution of the azide (193 mg) in THF (8.8 ml) and water (1.8 ml) is added with a 1M solution of Me₃P in THF (0.74 ml). The reaction solution is stirred at room temperature for 2 hours up to completion. The solvent is evaporated under reduced pressure, and the crude amine (VIII a) is sufficiently pure to be used for the next step without further purification. ¹H-NMR (400 MHz, D₂O, δ-H): 1.47 (s, 3H, CH₃), 168 (s, 3H, CH₃), 3.40 (dd, 1H, H-5'), 3.49 (dd, 1H, H-5'), 4.38 (m, 1H, H-4'), 4.90 (dd, 1H, H-1'), 4.94 (d, 1H, H-3'), 5.05 (dd, 1H, H-2') 7.76 (s, 1H, H-6).

### Synthesis of (IX a)

A solution of the commercial dipeptide Gly-Asn (22 mg, 0.11 mmol) in dioxane (150 µL) and water (250 µL) is added with Na₂CO₃ (26.5 mg) and FmocCl (31 mg, 1.3 eq). The reaction is allowed to stir at room temperature for 16h, after which time it is complete. After the addition of water (5 ml), the solution is extracted with EtOAc (3 x 5 ml). The combined organic phases are then re-extracted with a saturated solution of NaHCO₃ (3 x 5 ml), and the combined aqueous extracts are acidified to pH 1 by addition of 1M HCl and then re-extracted with AcOEt (3 x 5 ml). The combined organic phases are dried over Na₂SO₄ and evaporated to dryness, thereby obtaining (IX a) in quantitative yield. ¹H-NMR (400 MHz, D₂O, δ-H): 1.98 (m, 1H, Asn-β), 2.18 (m, 1H, Asn-β), 2.33 (m, 2H, Asn-γ), 3.90 (m, 2H, Gly-α), 4.23 (m, 1H), 4.31 (m, 1H), 4.47 (dd, 1H, Asn-α), 7.31 (m, 2H, Ar), 7.38 (m, 2H, Ar), 7.69 (m, 2H, Ar), 7.81 (m, 2H, Ar).

### Synthesis of (IV)

Condensation of (IX a) with (VIII a): A solution of (IX a) (20 mg) and (VIII a) (30 mg, 1.1 eq) in anhydrous DMF (1.5 ml) is added with DCC (18 mg, 1.2 eq) and HOBT (19.5 mg, 2 eq), and the reaction solution is allowed to stir for 16h at room temperature. The DMF is evaporated under reduced pressure, and the crude product is used as is for the next step.

Deprotection of the Fmoc group: A solution of the crude condensate (12 mg) in DMF (800 µL) is added with piperidine (200 µL), and the reaction is stirred for 10 minutes at room temperature. The solvent is evaporated under reduced pressure, and the crude product is washed with dichloromethane (2x 5 ml) and then used for the subsequent step.

Guanylation: A solution of the Fmoc-deprotected condensate (22 mg) in MeOH (300 µL) is added with 3,5-dimethylpyrazole-1-carboxyamidine (45 mg, 10 eq), and the reaction is stirred for 16 hours at room temperature, followed by additional 6 hours at reflux. The solvent is then evaporated under reduced pressure, and the solid is washed with dichloromethane (2 x 10 ml) and then used for the next step. ¹H-NMR (400 MHz, D₂O-CD₃OD, δ-H): 1.33 (s, 3H, CH₃), 1.54 (s, 3H, CH₃), 2.01 (m, 1H, Asn-β), 2.17 (m, 1H, Asn-β), 2.37 (m, 2H, Asn-γ), .3.37 (m, 1H, H-5'), 3.65 (m, 1H, H-5'), 4.04 (s, 2H, Gly-α), 4.03 (m, 1H), 4.11 (m, 1H), 4.42 (m, 1H), 4.63 (m, 1H), 7.53 (s, 1H, H-6).

Deprotection of 2',3'-acetonide: A solution of the guanylated condensation product (17 mg) in 7:3 AcOH:H₂O (2 ml) is allowed to stir at room temperature for 16 hours and then heated to 50°C over 10 hours under argon. The solvent is then removed by evaporation under reduced pressure, and the solid is washed with dichloromethane (2 x 5 ml) and then with MeOH (2 ml). The white solid obtained is analyzed by HPLC, LC-MS, 1D- and 2D-NMR, and it is indistinguishable from that obtained from pseudouridimycin (II) by semi-synthesis as described in Example 5.

## Claims

1. Compounds of formula (I) 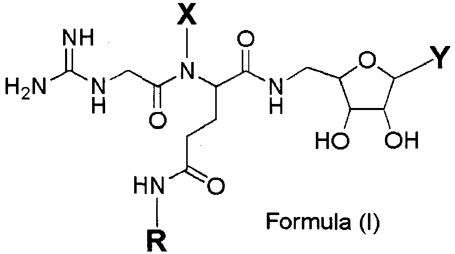
where R is independently selected from:
• H,
• a straight, branched, cyclic C₁-C₂₀ alkyl group, or combinations thereof,
• a straight, branched, cyclic C₂-C₂₀ alkenyl group, or combinations thereof;
• a straight, branched, cyclic C₂-C₂₀ alkynyl group, or combinations thereof;
• a benzylic group;
• a naphthylic group;
X is independently selected from H, OH, NH₂
Y is independently selected from one of the following heterocyclic groups:

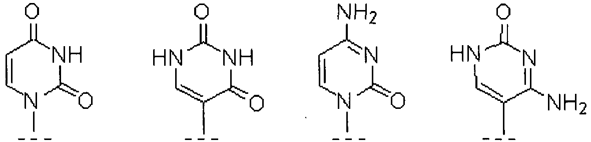

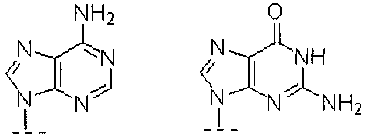

2. The compounds according to claim 1, **characterized in that** said R is H, said X is OH and said Y is 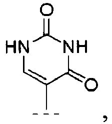 and the compound is represented by the formula (II)

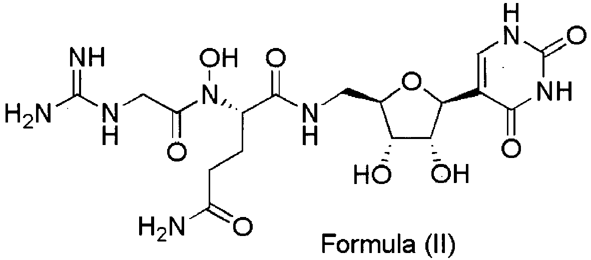

3. The compounds according to claim 1, **characterized in that** said X is selected to be equal to OH, said R is selected to be equal to PhCH₂-, said Y is selected to be equal to 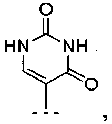 and the compound is represented by the formula (III)

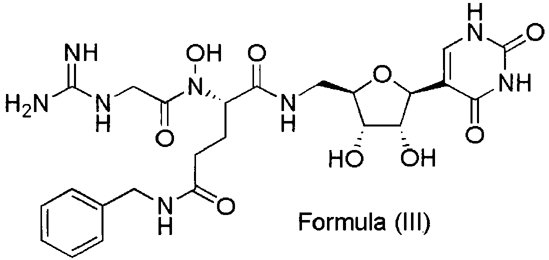

4. The compounds according to claim 1, **characterized in that** said X is selected to be equal to H, said R is selected to be equal to H, said Y is selected to be equal to 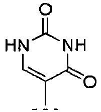 , and the compound is represented by the formula (IV)

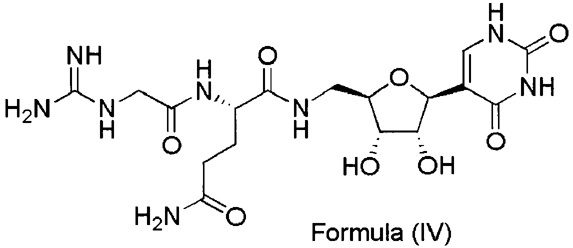

5. Compounds of formula (V)

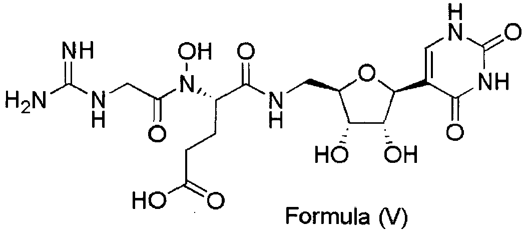

6. Micro-organism Streptomyces sp. NAI38640, deposited as number DSM26212 on July 20, 2012, in the Deutsche Sammlung von Mikroorganismen und Zellkulturen GmbH (DSMZ).

7. The compounds of formula (I) according to claim 1 for use as a medicament.

8. The compounds of formula (I) according to claim 1 for use in the treatment of infectious diseases.

9. A process for preparing the compounds of formula (I) of claim 1, **characterized in that** it comprises culturing the Streptomyces spp. NAI38640, deposited as number DSM26212 on July 20, 2012 in the Deutsche Sammlung von Mikroorganismen und Zellkulturen GmbH (DSMZ), or a variant thereof, or a mutant thereof, able to produce a compound of formula (II) 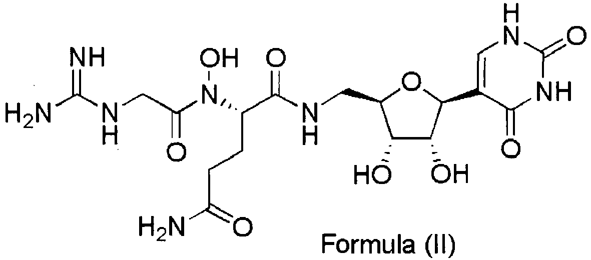 collecting the product of formula (II) from the mycelium and/or fermentation broth, isolating the pure compound of formula (II) through chromatographic techniques, lengthening the glutamine chain of compound (II) through hydrolysis of the primary amide and subsequent amidation with a suitable R-NH₂ group, and removing the N-hydroxylic group occurs through reduction with suitable reducing agents.

10. Pharmaceutical compositions comprising the compounds of formula (I) of claim 1 as active ingredients.

11. The pharmaceutical compositions according to claim 10 for use as a medicament.

12. The pharmaceutical compositions according to claim 10 for use in the treatment of infectious diseases.

## Patentansprüche

1. Verbindungen gemäß der Formel (I) 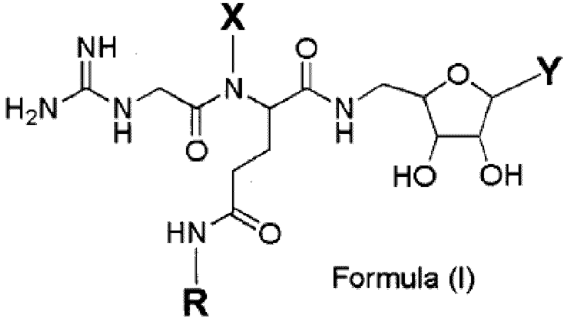
wobei R unabhängig ausgewählt ist aus:
• H,
• einer geraden, verzweigten, cyclischen C₁-C₂₀-Alkylgruppe oder Kombinationen davon,
• einer geraden, verzweigten, cyclischen C₂-C₂₀-Alkenylgruppe oder Kombinationen davon;
• einer geraden, verzweigten, cyclischen C₂-C₂₀-Alkinylgruppe oder Kombinationen davon;
• einer Benzylgruppe;
• einer Naphthylgruppe;
X unabhängig ausgewählt ist aus H, OH, NH₂
Y unabhängig ausgewählt ist aus einer der folgenden heterocyclischen Gruppen:

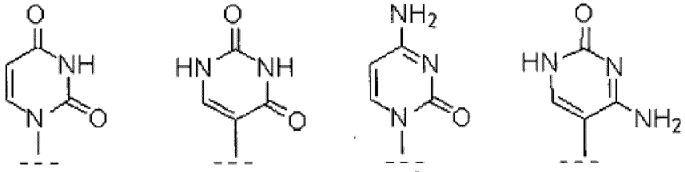

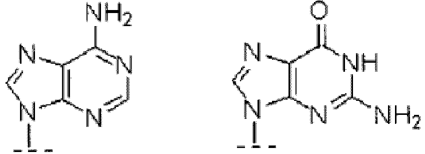

2. Die Verbindungen gemäß Anspruch 1, **dadurch gekennzeichnet, dass** R für H steht, X für OH steht und Y für 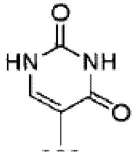 steht, und die Verbindung durch die Formel (II) 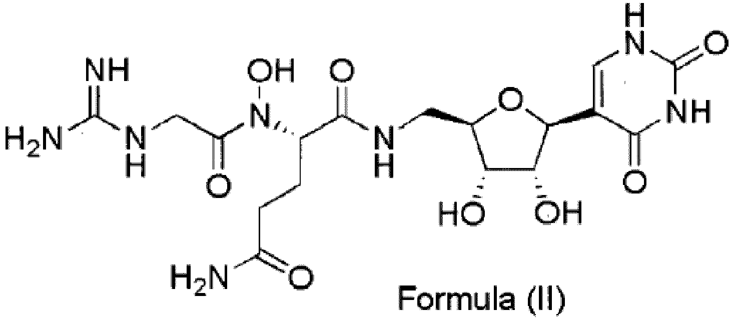 dargestellt wird.

3. Die Verbindungen gemäß Anspruch 1, **dadurch gekennzeichnet, dass** X ausgewählt wird, dass es gleich OH ist, R ausgewählt wird, dass es gleich PhCH₂ ist, Y ausgewählt wird, dass es gleich 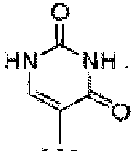 ist, und wobei die Verbindung durch die Formel (III) 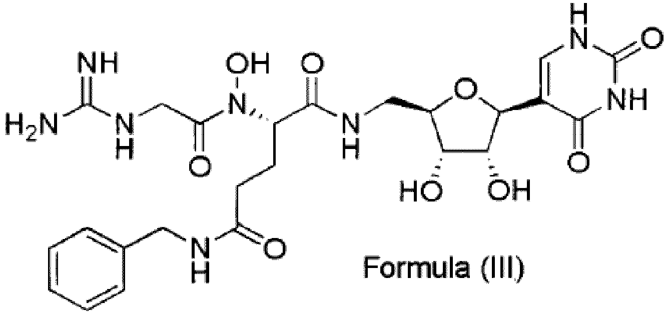 dargestellt wird.

4. Die Verbindungen gemäß Anspruch 1, **dadurch gekennzeichnet, dass** X ausgewählt wird, dass es gleich H ist, R ausgewählt wird, dass es gleich H ist, und Y ausgewählt wird, dass es gleich 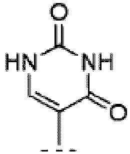 ist, und die Verbindung durch die Formel (IV) 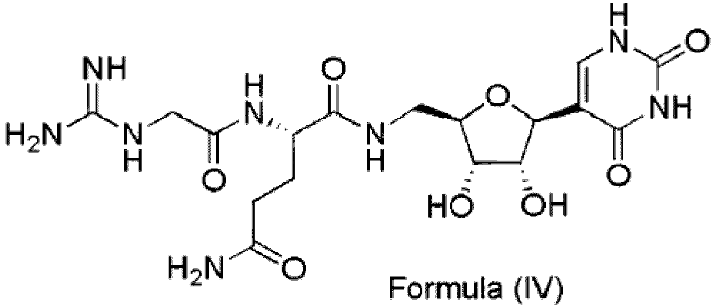 dargestellt wird.

5. Verbindungen der Formel (V)

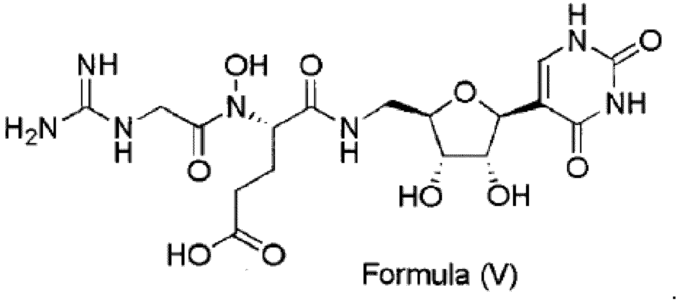

6. Mikroorganismus Streptomyces sp. NAI38640, hinterlegt unter der Nummer DSM26212 am 20. Juli 2012 bei der Deutschen Sammlung von Mikroorganismen und Zellkulturen GmbH (DSMZ).

7. Die Verbindungen der Formel (I) gemäß Anspruch 1 zur Verwendung als Arzneimittel.

8. Die Verbindungen der Formel (I) gemäß Anspruch 1 zur Verwendung bei der Behandlung von Infektionskrankheiten.

9. Ein Verfahren zur Herstellung der Verbindungen der Formel (I) gemäß Anspruch 1, **dadurch gekennzeichnet, dass** es die Kultivierung des Streptomyces spp. NAI38640 umfasst, hinterlegt unter der Nummer DSM26212 am 20. Juli 2012 bei der Deutschen Sammlung von Mikroorganismen und Zellkulturen GmbH (DSMZ), oder einer Varianten davon, oder einer Mutanten davon, der in der Lage ist, eine Verbindung der Formel (II) zu erzeugen 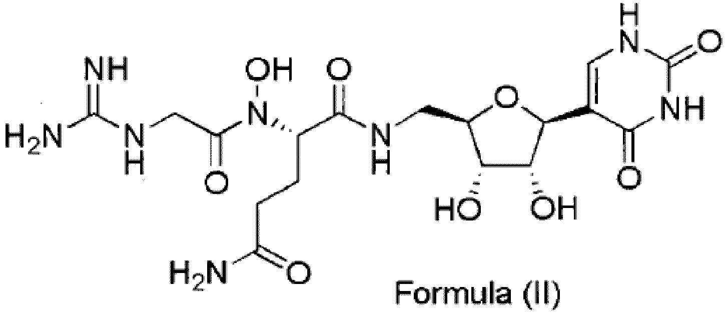 Gewinnen des Produktes der Formel (II) aus dem Myzel- und/oder der Fermentationsbrühe, Isolieren der reinen Verbindung der Formel (II) durch chromatographische Techniken, Verlängern der Glutaminkette der Verbindung (II) durch Hydrolyse des primären Amids und anschließendes Amidieren mit einer geeigneten R-NH₂-Gruppe, und Entfernen der N-Hydroxylgruppe durch Reduktion mit geeigneten Reduktionsmitteln.

10. Pharmazeutische Zusammensetzungen enthaltend die Verbindungen der Formel (I) gemäß Anspruch 1 als Wirkstoff.

11. Die pharmazeutischen Zusammensetzungen gemäß Anspruch 10 zur Verwendung als Arzneimittel.

12. Die pharmazeutischen Zusammensetzungen gemäß Anspruch 10 zur Verwendung bei der Behandlung von Infektionskrankheiten.

## Revendications

1. Composés de formule (I) 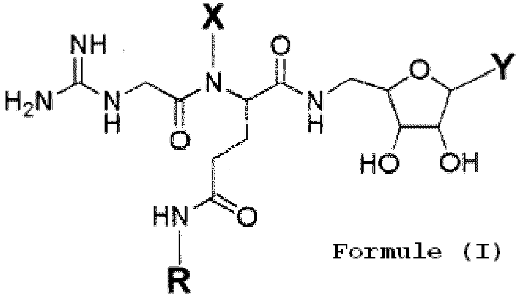
où R est choisi indépendamment parmi :
H,
un groupe alkyle cyclique en C₁ à C₂₀ linéaire, ramifié, ou leurs combinaisons,
un groupe alcényle cyclique en C₂ à C₂₀ linéaire, ramifié, ou leurs combinaisons ;
un groupe alcynyle cyclique en C₂ à C₂₀ linéaire, ramifié, ou leurs combinaisons ;
un groupe benzylique ;
un groupe naphtylique ;
X est choisi indépendamment parmi H, OH, NH₂
Y est choisi indépendamment parmi l'un des groupes hétérocycliques suivantes :

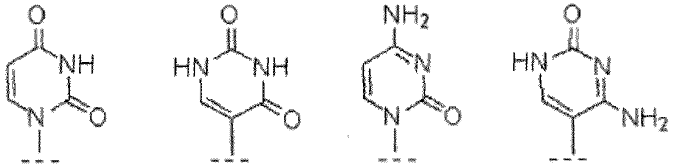

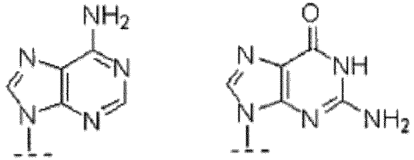

2. Composés selon la revendication 1, **caractérisés en ce que** ledit R représente H, ledit X représente OH et ledit Y représente 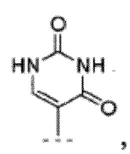 et le composé est représenté par la formule (II)

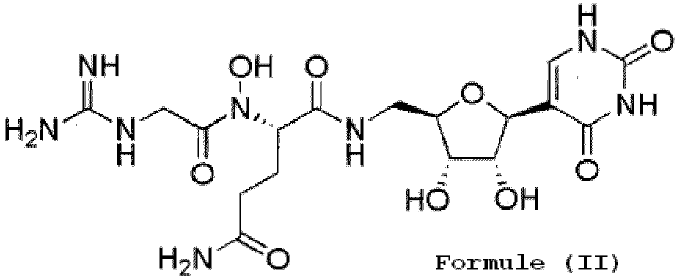

3. Composés selon la revendication 1, **caractérisés en ce que** ledit X est choisi pour être égal à OH, ledit R est choisi pour être égal à PhCH₂-, ledit Y est choisi pour être égal à 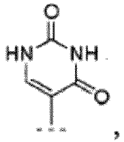 et le composé est représenté par la formule (III)

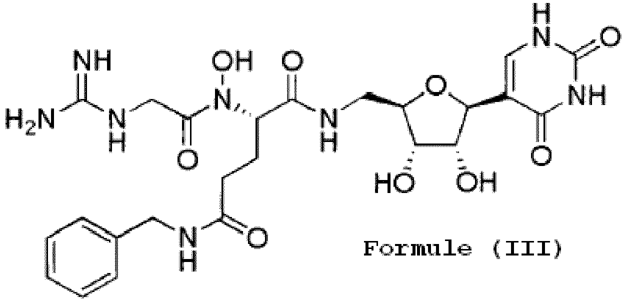

4. Composés selon la revendication 1, **caractérisés en ce que** ledit X est choisi pour être égal à H, ledit R est choisi pour être égal à H, ledit Y est choisi pour être égal à 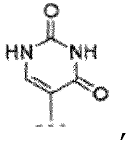 et le composé est représenté par la formule (IV)

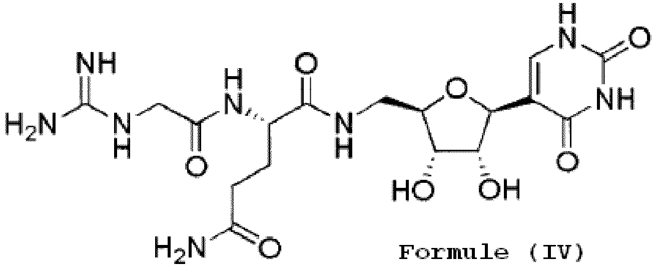

5. Composés de formule (V)

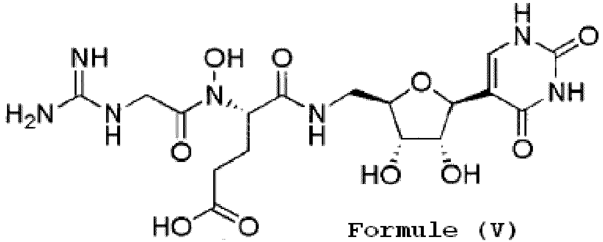

6. Micro-organisme Streptomyces sp. NAI38640, déposé sous le numéro DSM26212 le 20 juillet 2012, à la Deutsche Sammlung von Mikroorganismen und Zellkulturen GmbH (DSMZ).

7. Composés de formule (I) selon la revendication 1 pour une utilisation en tant que médicament.

8. Composés de formule (I) selon la revendication 1 pour une utilisation dans le traitement de maladies infectieuses.

9. Procédé de préparation des composés de formule (I) selon la revendication 1, **caractérisé en ce qu'**il comprend la culture du Streptomyces spp. NAI38640, déposé sous le numéro DSM26212 le 20 juillet 2012, à la Deutsche Sammlung von Mikroorganismen und Zellkulturen GmbH (DSMZ), ou de l'un de ses variants, ou de l'un de ses mutants, capable de produire un composé de formule (II) 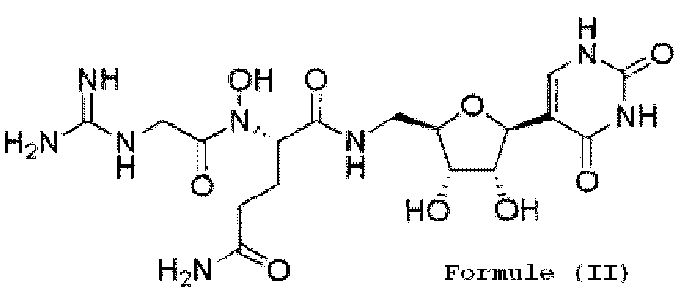 le recueil du produit de formule (II) à partir du mycélium et/ou du bouillon de fermentation, l'isolement du composé pur de formule (II) à l'aide de techniques chromatographiques, l'allongement de la chaîne de glutamine du composé (II) à l'aide de l'hydrolyse de l'amide primaire et de l'amidation subséquente avec un groupe R-NH₂ approprié, et l'élimination du groupe N-hydroxylique se produit à l'aide d'une réduction avec des agents réducteurs appropriés.

10. Compositions pharmaceutiques comprenant les composés de formule (I) selon la revendication 1 en tant que principes actifs.

11. Compositions pharmaceutiques selon la revendication 10 pour une utilisation en tant que médicament.

12. Compositions pharmaceutiques selon la revendication 10 pour une utilisation dans le traitement de maladies infectieuses.


### SEQUENCE LISTING

<110> NEW ANTI-INFECTIVES CONSORTIUM S.c.a.r.l
<120> PSEUDOURIDIMICINA (PUM) E SUOI DERIVATI
<130> FT/PV/ac 12431
<160> 1
<170> PatentIn version 3.5
<210> 1
   <211> 1441
   <212> DNA
   <213> Streptomyces sp. DSM 26212
<400> 1
   aacgctggcg gcgtgcttaa cacatgcaag tcgaacgatg aacctccttc gggaggggat      60
   tagtggcgaa cgggtgagta acacgtgggc aatctgccct tcactctggg acaagccctg     120
   gaaacggggt ctaataccgg atacgaccac cgaccgcatg gtctggtggt ggaaagctcc     180
   ggcggtgaag gatgagcccg cggcctatca gcttgttggt ggggtgatgg cctaccaagg     240
   cgacgacggg tagccggcct gagagggcga ccggccacac tgggactgag acacggccca     300
   gactcctacg ggaggcagca gtggggaata ttgcacaatg ggcgaaagcc tgatgcagcg     360
   acgccgcgtg agggatgacg gccttcgggt tgtaaacctc tttcagcagg gaagaagcga     420
   aagtgacggt acctgcagaa gaagcgccgg ctaactacgt gccagcagcc gcggtaatac     480
   gtagggcgca agcgttgtcc ggaattattg ggcgtaaaga gctcgtaggc ggcttgtcac     540
   gtcggatgtg aaagcccggg gcttaacccc gggtctgcat tcgatacggg caggctagag     600
   ttcggtaggg gagatcggaa ttcctggtgt agcggtgaaa tgcgcagata tcaggaggaa     660
   caccggtggc gaaggcggat ctctgggccg atactgacgc tgaggagcga aagcgtgggg     720
   agcgaacagg attagatacc ctggtagtcc acgccgtaaa cgttgggaac taggtgtggg     780
   cgacattcca cgtcgtccgt gccgcagcta acgcattaag ttccccgcct ggggagtacg     840
   gccgcaaggc taaaactcaa aggaattgac gggggcccgc acaagcagcg gagcatgtgg     900
   cttaattcga cgcaacgcga agaaccttac caaggcttga catacaccgg aaaaccctgg     960
   agacagggtc ccccttgtgg tcggtgtaca ggtggtgcat ggctgtcgtc agctcgtgtc    1020
   gtgagatgtt gggttaagtc ccgcaacgag cgcaaccctt gttctgtgtt gccagcatgc    1080
   ccttcggggt gatggggact cacaggagac tgccggggtc aactcggagg aaggtgggga    1140
   cgacgtcaag tcatcatgcc ccttatgtct tgggctgcac acgtgctaca atggccggta    1200
   caatgagctg cgataccgcg aggtggagcg aatctcaaaa agccggtctc agttcggatt    1260
   ggggtctgca actcgacccc atgaagtcgg agttgctagt aatcgcagat cagcattgct    1320
   gcggtgaata cgttcccggg ccttgtacac accgcccgtc acgtcacgaa agtcggtaac    1380
   acccgaagcc ggtggcccaa ccccttgtgg gagggaatcg tcgaaggtgg gactggcgat    1440
   t                                                                    1441